(19) Europäisches Patentamt
European Patent Office
Office européen des brevets

(11) EP 3 370 752 B1

(12) EUROPEAN PATENT SPECIFICATION

(45) Date of publication and mention of the grant of the patent:
**09.10.2024 Bulletin 2024/41**

(21) Application number: **16862533.3**

(22) Date of filing: **02.11.2016**

(51) International Patent Classification (IPC):
***A61K 36/68*** *(2006.01)* ***A23K 10/30*** *(2016.01)*

(52) Cooperative Patent Classification (CPC):
**A23K 10/30; A61K 36/68;** Y02P 60/21

(86) International application number:
**PCT/NZ2016/050175**

(87) International publication number:
**WO 2017/078544 (11.05.2017 Gazette 2017/19)**

# (54) NUTRIENT LOSS REDUCTION METHOD

NÄHRSTOFFVERLUSTVERRINGERUNGSVERFAHREN

PROCÉDÉ DE RÉDUCTION DE LA PERTE DE NUTRIMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.11.2015 NZ 15713849**

(43) Date of publication of application:
**12.09.2018 Bulletin 2018/37**

(73) Proprietor: **PGG Wrightson Seeds Limited**
**3204 Hamilton (NZ)**

(72) Inventors:
- **JUDSON, Howard Glenn**
  **3204 Hamilton (NZ)**
- **EDWARDS, Grant Raymond**
  **3204 Hamilton (NZ)**
- **BARRELL, Graham Keith**
  **3204 Hamilton (NZ)**

(74) Representative: **Boult Wade Tennant LLP**
**Salisbury Square House**
**8 Salisbury Square**
**London EC4Y 8AP (GB)**

(56) References cited:
**WO-A1-2017/065619**

- **RUMBALL W ET AL: "'Grasslands Lancelot" plantain (Plantago lanceolata L.)", NEW ZEALAND JOURNAL OF AGRICULTURAL RESEARCH, vol. 40, no. 3, 1997, pages 373 - 377, XP002790412, ISSN: 0028-8233**
- **BELANGER G ET AL: "Species and chlorine fertilisation affect dietary cation-anion difference of cool-season grasses", XX INTERNATIONAL GRASSLAND CONGRESS: OFFERED PAPERS WAGENINGEN ACADEMIC PUBLISHERS, POSTBUS 220, 6700 AE WAGENINGEN, NETHERLANDS, 2005, & 20TH INTERNATIONAL GRASSLAND CONGRESS; DUBLIN, IRELAND; JUNE 26 -JULY 01, 2005, pages 276, XP002790413**
- **H G JUDSON ET AL: "Benefits and uses of plantain (Plantago lanceolata) cv. Ceres Tonic in livestock production systems in New South Wales", PROCEEDINGS OF THE 26TH ANNUAL CONFERENCE OF THE GRASSLAND SOCIETY OF NSW, 1 January 2011 (2011-01-01), pages 151 - 152, XP055378150, Retrieved from the Internet <URL:http://grasslandnsw.com.au/FreeContent/2011/Judson Moorehead 2011.pdf> [retrieved on 20170602]**
- **CA GARDINER ET AL: "Potential for forage diet manipulation in New Zealand pasture ecosystems to mitigate ruminant urine derived N 2 O emissions: a review", NEW ZEALAND JOURNAL OF AGRICULTURAL RESEARCH., vol. 59, no. 3, 27 June 2016 (2016-06-27), pages 301 - 317, XP055558741, ISSN: 0028-8233, DOI: 10.1080/00288233.2016.1190386**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- BOX L A ET AL: "Milk production and urinary nitrogen excretion of dairy cows grazing perennial ryegrass-white clover and pure plantain pastures", PROCEEDINGS OF THE NEW ZEALAND SOCIETY OF ANIMAL PRODUCTION, vol. 76, 4 July 2016 (2016-07-04), pages 18 - 21, XP002790414, ISSN: 0370-2731
- NAVARRETE SOLEDAD ET AL: "Bioactive compounds, aucubin and acteoside, in plantain (Plantago lanceolataL.) and their effect onin vitrorumen fermentation", ANIMAL FEED SCIENCE AND TECHNOLOGY, ELSEVIER, AMSTERDAM, NL, vol. 222, 19 October 2016 (2016-10-19), pages 158 - 167, XP029817081, ISSN: 0377-8401, DOI: 10.1016/J.ANIFEEDSCI.2016.10.008
- O'CONNELL C A ET AL: "Sustained diuretic effect of plantain when ingested by sheep", PROCEEDINGS OF THE NEW ZEALAND SOCIETY OF ANIMAL PRODUCTION, vol. 76, 4 July 2016 (2016-07-04), pages 14 - 17, XP002790415, ISSN: 0370-2731
- PEMBERTON, KG: "Response of the DCAD of plantain to potassium fertilisation.", PROCEEDINGS OF THE 17TH ASA CONFERENCE, Hobart Australia, XP 055381937, Retrieved from the Internet <URL:www.agronomy2015.com.au.>
- WOOWARD S.L: "Can diverse pasture mixtures reduce nitrogen losses?", PROCEEDINGS OF THE 5TH AUSTRALASIAN SCIENCE SYMPOSIUM 2012, pages 463 - 464, XP009510497
- EDWARDS G.R ET AL.: "Milk Production and urination behaviour of dairy cows grazing diverse and simple pastures''.", PROCEEDINGS OF THE NEW ZEALAND SOCIETY OF ANIMAL PRODUCTION, vol. 75, 2015, pages 79 - 83, XP 055381939

**Description**

## FIELD OF THE INVENTION

The invention relates to methods, uses and products for reducing the loss of nutrients following excretion of those nutrients by an animal.

In particular the invention relates to methods, uses and plant material for reducing the rate at which nitrogenous compounds excreted in the urine of an animal are lost from practical utility. Mechanisms of loss may involve nitrification and possibly denitrification to produce compounds that can be leached and/or released to the atmosphere as gaseous compounds. This invention has particular application to domesticated animals, particularly sheep and cows, although this should not be seen as limiting.

## BACKGROUND TO THE INVENTION

An inevitable consequence of systems that farm animals is the production of faeces and urine both of which contain nitrogen (N).

Farmed animals excrete approximately 75% of the nitrogen that they ingest in their urine (50%) and faeces (25%). The nitrogen contained in excreted urine is generally in the form of urea, which is readily broken down to provide ammonia/ammonium by, for example, urease present in the soil. Nitrification of the ammonium initially produces nitrite, which undergoes further nitrification to produce nitrate. High nitrate can be problematic because it is not retained by soil particles and readily leaches into groundwater before it can be utilised for plant growth. In some cases, such as under anaerobic conditions, the nitrate can denitrify to produce nitrogen gas, nitrous oxide, nitric oxide and ammonia, which in gaseous forms can be released to the air.

The risk of leaching nitrogenous products (particularly nitrate) into groundwater is especially acute in porous soils, such as gravels and after heavy rainfall events or in continually wet soils where water moves through the profile taking nitrogen below the root zone, where it could otherwise be utilised for plant growth.

Nitrogen is an essential macronutrient for pastures and any loss of nitrogen from the farm system must be accounted for primarily through the application of fertiliser or legume-Rhizobia fixation.

Where the groundwater serves as a source of drinking water, excessive nitrate levels have been linked with the infant disease commonly known as "blue baby syndrome" (methemoglobinemia) - particularly in bottle-fed babies younger than 4 months of age.

There are a number of methods that have been adopted to decrease the amount of nitrogen which enters the groundwater in farming regions, including the use of specific plant species which readily uptake nitrogen (as ammonium and/or nitrate) from the soil. One problem with relying on this technique is that as animal stocking levels increase, the plants cannot utilise enough of the excreted nitrogen to prevent leaching into the groundwater.

Another method employs the use of pasture species that, themselves, contain reduced levels of nitrogen. The urine produced by animals that consume these pasture species has a reduced level of nitrogen. At present, however, very few viable low-nitrogen plant species are available.

Another method to decrease groundwater pollution is to use stand-off pads which are areas that the animals are herded into to further digest the consumed herbage. These pads may comprise a hard (such as concrete) or soft (such as woodchip) surface and the animal excrement is contained and may be collected and further directed into storage ponds. A significant problem associated with this widely-used approach is the cost of building the infrastructure needed to collect, store and distribute the excrement.

A still further method to decrease nitrate leaching which has received recent interest is the use of a chemical nitrification inhibitor which is typically sprayed onto the pasture prior to animals being allowed to graze the area. Both dicyandiamide (DCD) and 3,4-dimethlpyrazole phosphate (DMPP) slow the conversion of ammonium to nitrite by *Nitrosomonas spp.* and therefore limit the amount of nitrate being produced. Despite no widely recognised standard for an acceptable level of DCD in milk, the widespread use of DCD has been curtailed by the discovery of low levels in milk products produced by cows grazed in areas in which DCD has been sprayed. Furthermore, with a growing trend towards organic production, the use of chemical nitrification inhibitors in the organic farming sector is becoming less popular.

There is a need for a farm management practice which leads to a reduction in nutrient leaching, particularly nitrate leaching, which addresses one or more of the foregoing problems or at least provides the public with a useful choice.

## SUMMARY OF THE INVENTION

In a first aspect the invention provides a method of increasing the urine volume of an animal, the method including the step of administering plant material derived from a plant of the genus *Plantago* to the animal. The invention also provides the use of plant material derived from a plant of the genus *Plantago* to increase the urine volume of an animal.

In a second aspect the invention provides a method of causing diuresis in an animal, the method including the step of administering plant material derived from a plant of the genus *Plantago* to the animal. The invention also provides the use of plant material derived from a plant of the genus *Plantago* to cause diuresis in an animal.

The inventors have now determined that when animals are fed plant material derived from a plant of the genus *Plantago,* the urine volume of the animal increases significantly. This increase in urine volume may be observed in a number of different ways including more frequent urination and/or larger urine patches, both of which can have the effect of distributing the animal's daily urinary excretion of dissolved components over a larger ground area. Larger urine patches may be the result of the animal moving while urinating and/or where the soil is prone to at least partially disperse the urine across its surface rather than instantly allow it to absorb into the soil. By distributing the dissolved components of the urine over a larger area it is believed that the dissolved components, or derivatives thereof, are able to be more readily utilised by growing plant material (eg grass) before leaching below the root zone. As such, the increase in urine volume is believed to result in a greater proportion of the nitrogenous waste that is excreted in the urine being made available to ground cover for plant uptake.

Provided herein is furthermore a method of reducing the pH of the urine of an animal, the method including the step of administering plant material derived from a plant of the genus *Plantago* to the animal. The invention also provides the use of plant material derived from a plant of the genus *Plantago* to reduce the pH of the urine of an animal.

The inventors have now determined that when animals are fed plant material derived from a plant of the genus *Plantago,* the pH of the urine of the animal decreases significantly. This decrease in urine pH is believed to result in a smaller proportion of the nitrogenous waste that is excreted in the urine being lost through leaching and/or conversion to gaseous compounds. The decrease in urine pH is believed to result in a greater proportion of the nitrogenous waste that is excreted in the urine being made available to ground cover for plant uptake.

Animals typically excrete nitrogenous waste in the urine in the form of urea. Rapid hydrolysis of urea, typically in the presence of endogenous urease, provides ammonia ($NH_3$) which of itself may be lost as a gas and ammonium ($NH_4^+$ - the protonated form of ammonia). Ammonia exists in aqueous environments in equilibrium with ammonium.

In the presence of endogenous soil bacteria, ammonium-N is oxidised to nitrite and further to nitrate:

$$2\ NH_4^+ + 3\ O_2 \rightleftharpoons 2\ NO_2^- + 2\ H_2O + 4\ H^+ \qquad (\textit{Nitrosomonas}\ \text{spp.})$$

$$2\ NO_2^- + O_2 \rightleftharpoons 2\ NO_3^- \qquad (\textit{Nitrobacter}\ \text{spp.},\ \textit{Nitrospina}\ \text{spp.})$$

Without wishing to be bound by theory, it is now believed that the decrease in urine pH shifts the nitrite-producing equilibrium to the left, because of the increased supply of protons, thereby slowing the rate of nitrate production from available nitrite. By slowing the rate of nitrate production, it is believed that plant groundcover is able to uptake a greater proportion of available nitrogenous products before the nutrients are leached below the root depth. Similarly, where denitrification occurs, it is believed that the rate at which gaseous products are formed can be reduced thereby allowing plant uptake of nitrogenous products to occur more efficiently. It is believed that the net result of this reduction in the rate of nitrate production is that the amount of excreted nitrogenous waste that is leached and/or released to the atmosphere as gaseous compounds is reduced.

In a fourth aspect the invention provides a process of screening a population of plants of the genus *Plantago* for use in increasing the urine volume of an animal, the process including the steps of:

a. measuring the DCAD of each of the population of plants;

b. selecting one or more of the population of plants having DCAD less than 10 meq/100 g DM.

In a fifth aspect the invention provides a process of screening a population of plants of the genus *Plantago* for use in reducing the pH of the urine of an animal, the process including the steps of:

a. measuring the DCAD of each of the population of plants;

b. selecting one or more of the population of plants having DCAD less than 10 meq/100 g DM.

Without wishing to be bound by theory, the inventors believe that plants of the genus *Plantago* possessing a DCAD value of less than 10 meq/100 g DM are responsible for significantly increasing the urine volume of an animal

and/or significantly reducing the pH of the urine of an animal.

In a sixth aspect the invention provides a population of plants of the genus *Plantago* having DCAD less than 10 meq/100 g DM. Such plants are particularly suited to those circumstances when used in increasing the urine volume of an animal and/or for use in reducing the pH of the urine of an animal.

Increasing the urine volume of an animal is believed to result in a greater proportion of the nitrogenous waste that is excreted in the urine being made available to ground cover for plant uptake. Reducing the pH of the urine of an animal is believed to reduce the rate at which nitrogenous compounds excreted in the urine of the animal undergo nitrification and possibly denitrification to produce compounds that can be leached and/or released to the atmosphere as gaseous compounds.

Furthermore provided herein is a method of conditioning a population of plants of the genus *Plantago* for use in increasing the urine volume of an animal and/or for use in reducing the pH of the urine of an animal.

Also described herein is a population of plants of the genus *Plantago* conditioned for use in increasing the urine volume of an animal and/or for use in reducing the pH of the urine of an animal.

Described is also a method of reducing the amount of nitrate derived from an animal that is leached into groundwater and/or surface water, the method including the step of administering plant material derived from a plant of the genus *Plantago* to the animal.

Additionally provided herein is a method of reducing nitrogenous gases derived from an animal, the method including the step of administering plant material derived from a plant of the genus *Plantago* to the animal.

Further provided herein is a method of reducing urinary nitrogen concentration in an animal, the method including the step of administering plant material derived from a plant of the genus *Plantago* to the animal.

Described herein is also a method of reducing nitrate levels in surface water and/or groundwater in the vicinity of an animal, the method including the step of administering plant material derived from a plant of the genus *Plantago* to the animal.

Also described herein is a method of reducing nitrogen loading in individual urine patches derived from an animal, the method including the step of administering plant material derived from a plant of the genus *Plantago* to the animal.

Provided is furthermore a method of reducing nitrogen volatilisation from a pastoral system, the method including the steps of:

a) growing a plant of the genus *Plantago* in the pastoral system; and
b) administering plant material derived from the plant of the genus *Plantago* to the animal.

Furthrmore described is a method of reducing greenhouse gas emissions from a pastoral system, the method including the steps of:

a) growing a plant of the genus *Plantago* in the pastoral system; and
b) administering plant material derived from the plant of the genus *Plantago* to the animal.

Finally also provided herein is a method of reducing nitrate leaching from a pastoral system, the method including the steps of:

a) growing a plant of the genus *Plantago* in the pastoral system; and
b) administering plant material derived from the plant of the genus *Plantago* to the animal.

## BRIEF DESCRIPTION OF DRAWING

Further aspects will become apparent from the following description which is given by way of example only and with reference to the accompanying drawing in which:

Figure 1 Figure 1 depicts a line graph comparing urine pH over time for sheep fed ryegrass or plantain (Tonic), and showing that urine pH was significantly reduced when fed plantain.

## DETAILED DESCRIPTION OF THE INVENTION

As used herein "animal" includes reference to domesticated farm animals, including mammals. Examples of such mammals are cattle, horses, sheep, goats and pigs. Preferably the present invention is for use in reducing the pH of urine from ruminant animals (such as cattle, goats, sheep, yaks, deer) - particularly cattle and sheep. Still more preferably the present invention is for use in reducing the pH of urine from dairy cattle and sheep. Dairy cows are most preferred as they form the largest part of the dairy herd and are believed to contribute the most to nitrate leaching.

Plants of the genus *Plantago* are not commonly used as forage crops, and are often thought of as weeds. Because of this, in many countries plants of the genus *Plantago* are eradicated from pasture systems. A preferred species of the genus *Plantago,* commonly referred to as plantain, that may be used in the present invention is *Plantago lanceolata.* Preferably a plantain having a Mediterranean genetic origin is used. Preferably the plantain used is characterised as providing winter growth, although some winter dormant cultivars (WDA+) are effective. A particularly preferred example of a plantain is *Plantago lanceolata* cv. Ceres Tonic, or breeding lines largely derived from that breeding pool eg. PG742.

Aspects of the present invention use plant material derived from a plant of the genus *Plantago.* As used herein the expression "plant material" includes reference to:

(a) plant growing *in situ* at the time of grazing. For example, the feed may be at least part of a forage crop that is eaten by grazing livestock as pasture or as crop residue (such as the residue left after a crop is harvested);

(b) propagating material of the plant of the genus *Plantago*;

(c) harvested material of the plant of the genus *Plantago*; and

(d) products obtained from harvested material of the plant of the genus *Plantago.*

Such products obtained from harvested material include material that has been conditioned into, for example, balage or conserved feed including, but not limited to, silage, balage, hay or haylage.

As used herein the expression "administering plant material derived from a plant of the genus *Plantago* to the animal" typically refers to any method of administering plant material derived from a plant of the genus *Plantago* to the animal orally, typically ingesting the plant material derived from a plant of the genus *Plantago* to the animal so that is enters the rumen in those cases where the animal is a ruminant. In its simplest form the plant material derived from a plant of the genus *Plantago* may be administered as part of the animal feed.

It is believed that any dosage of the plant material derived from a plant of the genus *Plantago* should produce a meaningful decrease in the pH of the urine of the animal and/or an increase in urine volume of the animal. In light of the buffering capacity of urine, the reduction in urine pH is more pronounced at elevated dosages of the plant material. Such dosages are conveniently provided as dry matter equivalent per liveweight per day. By way of example, the dosage may be 0.1-1 kg dry matter per day for a 40 kg sheep which equates to 0.0025-0.025 kg/kg liveweight per day. By way of further example, for a 250 kg yearling the dosage may be 1-10 kg dry matter per day (such as 5 kg dry matter per day) which equates to about 0.004-0.04 kg/kg liveweight per day (such as 0.02 kg/kg/day). Such dosages may be suitable for any of the animals contemplated by the present invention, including sheep and cattle.

As used herein the term "increasing", "increase" (as the context requires) with respect to a parameter refers to the modulation of the level of that parameter compared to the level of that parameter in the absence of the modulation. For example with respect to urine volume, "increasing", "increase" (as the context requires) refers to a modulation of the urine volume with respect to that level that would otherwise normally be found. For example, the increase in the urine volume of an animal by administering plant material derived from a plant of the genus *Plantago* to the animal may be determined with respect to:

a) the urine volume of the animal during a period prior to, or subsequent to, the period of time during which the plant material derived from a plant of the genus *Plantago* is administered to the animal; and/or
b) a control animal (or population of animals) that have not been administered with plant material derived from a plant of the genus *Plantago.*

Such a comparison can be made with respect to the total volume of urine excreted measured in a number of different ways, including:

a) the amount of urine excreted in a given time period (such as 6, 12 or 24 hours); and/or
b) the amount of urine excreted per discharge.

Preferably the comparison will be made with respect to the total volume of urine excreted in 24 hours, otherwise referred to as the daily urine volume.

One method of establishing the baseline urine volume of an animal is to feed the animal a plant material derived from ryegrass. In order to provide a statistically meaningful baseline urine volume it may be appropriate to measure the urine volume over a plurality of time periods, such as a plurality of days. Such a sampling period may be over 3, 5, 7 or more days.

The extent of the increase may be measured as the percentage increase in urine volume compared with the untreated animal or a control animal (or population of animals). The percentage increase may be as little as 1%, however will typically be greater than 5%, such as greater than 10%. The extent of the increase may be as large as 60% or more. Typically the extent of the increase will be between 5 and 100%, such as between 10 and 80%.

When not consuming a diet including plant material derived from a plant of the genus *Plantago,* typical urinary pH in animals can range from 4.5 to 8.5. A renal buffering system is primarily responsible for maintaining a slightly acidic to slightly alkaline urinary pH in ruminants, such as 6.6 to 8.1, but more typically a neutral to slightly alkaline pH such as 7.5-8.0.

As used herein, the expression "reducing", "reduce" (as the context requires) with respect to a parameter refers to the modulation of the level of that parameter compared to the level of that parameter in the absence of the modulation.

For example, as used herein, the expression "reducing the pH" can be assessed in relative terms and includes a reduction in pH of the order of at least 0.1 pH, such as at least 0.2 pH or even 0.5 pH. In some embodiments, the reduction in pH will be at least 1.0 pH, or at least 2.0 pH. It will be understood that such a reduction will be relative to the pH of the urine of the animal that has not recently (such as within the past 5 or 10 days) consumed a significant quantity (if any) of plant material derived from a plant of the genus *Plantago.* The urine pH of such an animal that has not consumed a significant quantity (if any) of plant material derived from a plant of the genus *Plantago* is referred to herein as a baseline pH.

One method of establishing the baseline pH of an animal is to feed the animal a plant material derived from ryegrass. In order to provide a statistically meaningful baseline pH it may be appropriate to take a plurality of urine samples and determine the pH of each sample. Such samples may be taken each day for 3, 5, 7 or more days.

As can be seen from Figure 1, the pH of the animal population fed ryegrass varied over the course of 7 days of the experiment, between about 6.6 and about 8.1. In contrast, the pH of the animal population fed Tonic (a plant of the genus *Plantago*) varied over the course of 7 days of the experiment, between about 5.1 and about 6.1. On any given day the difference in urine pH between the animal population fed ryegrass and the animal population fed Tonic was of the order of between 1 pH (day 1) and at least 2 pH (days 2 and 6).

In further embodiments, the expression "reducing the pH" can be assessed in absolute, rather than relative terms. As disclosed herein, the urinary pH in ruminants is of the order of 6.6 to 8.1, such as 7.5-8.0. Furthermore, as disclosed herein, it is believed that reducing the pH of the urine of an animal below neutral, to create an acidic environment, significantly reduces the rate of nitrate production thereby reducing the amount of excreted nitrogenous waste that is leached and/or released to the atmosphere as gaseous compounds. Accordingly in some embodiments, the methods and uses of the invention reduce the pH of the urine may be reduced to below 7.0, such as below 6.6, below 6.1 or even below 5.5. These statements are particularly well suited to describing "reducing the pH" of a ruminant animal. Dietary Cation to Anion Difference (DCAD) is a measure of the balance between potassium (K), sodium (Na), chloride (Cl) and sulphur (S) in an animal feed and is expressed as milliequivalents per 100 g dry matter (meq/100 g DM) and is calculated using the following formula:

$$DCAD = (\%Na \times 43.5 + \%K \times 25.6) - (\%Cl \times 28.2 + \%S \times 62.5)$$

A review by Jean-Luc Riond ("Animal Nutrition and Acid Base Balance"; European Journal of Nutrition 40: 245-254 (2001)) states that "Typically diets fed to dry cows have a DCAD of about 100 to 250 mEq/kg of diet dry matter. Rodriguez-Suarez, L.A. (1998) "Peripartturient responses of cows fed varying dietary cation-anion differences and calcium contents prepartum" PhD dissertation of the Michigan State University, and Jones, M.L. (2006) "The use of dietary cation anion difference for the reduction of urine pH in goats", MSc disseration of Oklahoma State University, teach that lowering the DCAD of feed is effective in reducing the pH of urine. Addition of anions to the diet to reduce DCAD is limited because of problems with palatability of the anionic salt sources commonly used."

Nonetheless, it has now been discovered that plants of the genus *Plantago* possessing a DCAD value of less than 10 meq/100 g DM (such as less than 0) are responsible for significantly reducing the pH of the urine of an animal, so that it may be possible to reduce the rate at which nitrogenous compounds excreted in the urine of the animal undergo nitrification and possibly denitrification to produce compounds that can be leached and/or released to the atmosphere as gaseous compounds. It is believed that success can be achieved using plants of the genus *Plantago* in spite of the published view that they would suffer poor palatability.

As such the plant of the genus *Plantago* that is suitable for use in the present invention may have a DCAD less than 10 meq/100 g DM, such as below 0 meq/100 g DM, such as less than -2 meq/100 g dry matter, or less than -5 meq/100 g dry matter, or even less than -10 meq/100 g dry matter. It has been found that plants having such low DCAD values are surprisingly palatable to animals.

Without wishing to be bound by theory, it is believed that a lower DCAD value may provide a greater reduction in urine pH.

In order to select plants that are most suitable for reducing urine pH, the invention provides a process of screening a population of plants of the genus *Plantago* for use in reducing the pH of the urine of an animal, the process including the steps of:

a. measuring the DCAD of each of the population of plants;

b. selecting one or more of the population of plants having DCAD less than 10 meq/100 g DM.

This selection method allows the rapid screening of a plant population for use in reducing the pH of the urine of an animal without the need to conduct animal testing. In general, the screening method contemplated will involve one or more of the following steps:

1. Harvesting plant material;
2. Freezing the plant material;
3. Freeze drying the plant material;
4. Grinding the plant material;
5. Measuring the sodium, potassium, chloride and/or sulfate levels in the plant material; and
6. Calculating the DCAD value using the equation: DCAD = (%Na × 43.5 + °/K × 25.6) - (%Cl × 28.2 + %S × 62.5).

In a further aspect the invention provides a plant of the genus *Plantago* selected by the screening process outlined herein.

Where a given population of plants of the genus Plantago do not satisfy the selection criteria of having a DCAD less than 10 meq/100 g DM, it may be possible to condition the population for use in increasing the urine volume of an animal and/or for use in reducing the pH of the urine of an animal. The step of conditioning may involve exposing the population of plants to chloride and/or sulphur so that the plant uptakes chloride and/or sulphur. The chloride and/or sulphur may be derived from a fertiliser. Sulphur may be provided in elemental form or as sulphate. In preferred embodiments, the fertiliser will lead to a reduced DCAD value, and typically such a fertiliser will have a Conditioning Variable (CV) which is negative, where the CV of the fertiliser is calculated by applying the chemical composition of the fertiliser to the following equation:

$$CV = (\%Na \times 43.5 + \%K \times 25.6) - (\%Cl \times 28.2 + \%S \times 62.5).$$

Advantageously, the present invention provides the user with a method of readily providing a feeding animal with nutrition whilst simultaneously reducing the deleterious environmental impact of the animal. At its simplest, the method of the invention is able to be performed by grazing an animal on a pasture which includes plant material derived from a plant of the genus *Plantago,* such as grazing an animal on pasture which includes live stands of a plant of the genus *Plantago.*

In some embodiments the animal feed is derived from a pasture system which includes at least one plant of the genus *Plantago,* and optionally also includes at least one plant selected from one or more of the following: ryegrass *(Lolium* spp.), clover *(Trifolium* spp.), cocksfoot (*Dactylis glomerata),* chicory (*Cichorium intybus*), tall fescue *(Festuca arundinacea),* Kentucky bluegrass (*Poa pratensis),* birdsfoot trefoil (*Lotus corniculatus*)*,* lucerne (*Medicago sativa*)*,* and prairie grass (*Bromus willdenowii).*

In some embodiments the animal feed is derived from a pasture system which includes at least one plant of the genus *Plantago,* and optionally also includes at least one plant selected from between only one and four of the following: ryegrass *(Lolium* spp.), clover *(Trifolium* spp.), cocksfoot (*Dactylis glomerata*)*,* chicory (*Cichorium intybus*)*,* tall fescue (*Festuca arundinacea),* Kentucky bluegrass (*Poa pratensis*)*,* birdsfoot trefoil (*Lotus corniculatus*)*,* lucerne (*Medicago sativa*)*,* and prairie grass (*Bromus willdenowii*)*.*

In some embodiments the animal feed is derived from a pasture system which includes at least one plant of the genus *Plantago,* and optionally also includes at least one plant selected from between only one and three of the following: ryegrass (*Lolium* spp.), clover (*Trifolium* spp.), cocksfoot (*Dactylis glomerata*)*,* chicory (*Cichorium intybus),* tall fescue (*Festuca arundinacea),* Kentucky bluegrass (*Poa pratensis*)*,* birdsfoot trefoil (*Lotus corniculatus),* lucerne (*Medicago sativa),* and prairie grass (*Bromus willdenowii*)*.*

In some embodiments the animal feed is derived from a pasture system which includes at least one plant of the genus *Plantago,* and optionally also includes at least one plant selected from only one or two of the following: ryegrass (*Lolium* spp.), clover (*Trifolium* spp.), cocksfoot (*Dactylis glomerata*), chicory (*Cichorium intybus*), tall fescue (*Festuca arundinacea*), Kentucky bluegrass (*Poa pratensis*), birdsfoot trefoil (*Lotus corniculatus*), lucerne (*Medicago sativa*), and prairie grass (*Bromus willdenowii*)*.*

In some embodiments the animal feed is derived from a pasture system which includes at least one plant of the genus *Plantago,* and optionally also includes at least one plant selected from only one of the following: ryegrass (*Lolium* spp.), clover (*Trifolium* spp.), cocksfoot (*Dactylis glomerata),* chicory (*Cichorium intybus*), tall fescue (*Festuca arundinacea*), Kentucky bluegrass (*Poa pratensis*), birdsfoot trefoil (*Lotus corniculatus*), lucerne (*Medicago sativa*), and prairie grass (*Bromus willdenowii).*

Advantageously, plants of the genus *Plantago* allow for similar productivity to more diverse pasture systems, such that plants of the genus *Plantago* can be used as the predominant or even sole animal food source (H.G. Judson, R. McAnulty and R. Sedcole Proceedings of the New Zealand Grassland Association 71: 201-205 (2009)).

The present invention is particularly well suited to modulating the pH and/or urine volume of domesticated farm animals, including mammals. Examples of such mammals are cattle, horses, sheep, goats and pigs. Preferably the present invention is for use in modulating the pH and/or urine volume of ruminant animals (such as cattle, goats, sheep, yaks, deer) - particularly cattle and sheep. Still more preferably the present invention is for use in modulating the pH and/or urine volume of dairy cattle and sheep. Dairy cows are most preferred as they form the largest part of the dairy herd and are believed to contribute the most to nitrate leaching.

As used herein, the expression "in the vicinity of an animal", particularly with respect to reducing nitrate levels in surface water and/or groundwater, refers to the sphere of influence of the animal on nitrate levels in that environment. For example, it will be understood that the impact of the animal on nitrate levels in surface water and/or groundwater will most noticeably be felt downstream (surface water) or down-gradient (groundwater) and that effect will typically decay as a function of distance from the animal. Nonetheless the effect may be observed some kilometres from the animal, however typically the sphere of influence will extend the vicinity of the animal to less than 5 km, such as less than 1 km, such as less than 100 m from the animal. Most noticeably the effect of the invention herein will be observed within 5 m of where the animal has passed in any given day in which the plant material derived from a plant of the genus *Plantago* is consumed.

Nonetheless, it will also be appreciated that where standoff pads are used to collect urine from animals, and where the urine is subsequently dispersed as liquid fertiliser, the sphere of influence of the animal may extend to those fertilised areas where the animal may not have passed for a significant period of time, if at all. In those cases, the reduced nitrate levels achieved in that fertilised land could still justifiably be said to have benefited from the invention and accordingly would fall within the expression "in the vicinity of an animal", since those fertilised areas are typically on the same broader farm/pastoral system that the animal has inhabited.

## EXAMPLES

### EXAMPLE 1 - Sheep Study IA

#### *Experimental*

Animals used in this study were 6 month old Romney × Southdown ewe hoggets. Prior to the study they were grazed on predominantly ryegrass/white clover pasture near Lincoln, Canterbury, New Zealand (Kimihia Research Centre) then transported on 2 June 2015 to the nearby (5.2 km) Johnstone Memorial Laboratory of Lincoln University where they were held indoors for 7 days in individual metabolism crates without access to drinking water. The sheep were allocated to one of two treatment groups (n = 8) that were balanced for live weight (average 38 kg) and urine production (daily volume) and held in two separate rooms at the Johnstone Memorial Laboratory, with 4 animals from each group per room. Treatments were either plantain (*Plantago lanceolata, cv* Ceres Tonic) or perennial ryegrass *(Lolium perenne,* cultivars Base and Expo) offered at rate so that each sheep consumed 4 litres of water in their feed daily. Each day's individual sheep feed allowance was computed from the dry matter content of the previous day's feed to maintain the constant daily water allocation. Any refused feed was collected the next day and weighed, then discarded. All procedures involving these animals were approved by the Lincoln University Animal Ethics Committee.

*Urine and faeces*

Urine and faecal matter were collected separately into collection trays and buckets situated under the grating of each metabolism crate. To prevent cross-contamination, net separators (shade cloth stretched over a metal frame and fixed underneath the metabolism crates) were used to trap faeces and allow urine to pass through the mesh.

Urine was acidified to prevent nitrogen losses by adding 250 mL of 12% $H_2SO_4$ solution to the collection trays. Daily urine volume for each sheep was measured in a volumetric flask and a 70 mL sample was placed into a polypropylene container and stored in a freezer. The collection trays were rinsed with tap water, returned to each metabolism crate and left until a non-acidified (fresh) sample of (70 mL) urine could be collected. Acid was then placed in the tray for urine collected overnight. The pH of fresh urine was measured with a portable pH reader (Pocket ISFET pH meter S2K712,

ISFETCOM Co Ltd, Hidaka, Saitama-Prefecture, Japan) and its total solids was determined with a portable refractometer (Uricon, Atago USA Inc, Kirkland, WA, USA). Measurements of pH and refraction index were taken within an hour of the animal urinating. Fresh and acidified urine samples were bagged separately in plastic bags and stored frozen for subsequent analyses.

The frozen, fresh urine samples were later thawed for measurement of their osmolality (mmol/kg) with a vapour pressure osmometer (VAPRO 5520, Wescor Inc., Logan, Utah, USA) and multiplying this value by the daily urine volume provided an estimate of the total osmols excreted per day.

The metabolism crates were cleaned daily to collect all faecal matter and each sheep's daily faecal production was weighed. A small sample was set aside to be freeze dried for subsequent analysis. The rest was divided between two perforated plastic bags which were weighed and placed in 90°C ovens for 48 h to dry. Drying time depended on the size of the sample, therefore if a sample did not dry in the allocated time it was left for a further 48 h. Once dry, the bags were re-weighed and the dry matter percentage calculated (i.e. dry weight/wet weight × 100). This gave two values for faecal dry matter percentage (DM %) for each sheep's daily output. The mean of these two DM% values was applied to the total faecal weight to determine faecal water content.

### *Results*

The daily intake of water was similar for each group (3915 and 3858 mL for plantain and ryegrass, respectively), but there was less dry matter (506 g per day) in plantain than ryegrass (744 g per day).

Ewes consuming plantain had greater urine volumes than those on ryegrass (3760 vs 2965 mL per day) but had lower water content of their faeces (219 vs 375 mL per day). Water balance, measured as the difference between water consumed and the summed loss in urine and faeces, was -54 mL per day for plantain (i.e. a loss) and +518 mL per day for ryegrass (i.e. a net gain).

The faeces of ewes consuming plantain was drier than that from ewes on ryegrass (32.9% vs 27.6%). Urine pH of ewes on plantain was lower than that of ewes on ryegrass (5.57 vs 7.39).

### *Discussion*

*Urine volume*

In spite of similar volumes of water ingested, ewes consuming plantain had greater urinary volumes than those on ryegrass. These differences in urine volume cannot be explained by faecal loss of water or by respiratory losses from metabolism of the extra dry matter of ryegrass (see below). Thus, it is concluded that the consumption of plantain has produced a diuresis in these sheep.

The calculation of water balance does not include evaporative water loss (insensible from skin and respiratory losses). Much of the insensible loss would be expected to be very similar for the two groups. However, respiratory water loss is likely to be greater in ryegrass ewes that were metabolising about 200 more g per day of dry matter then the plantain ewes. Nevertheless, the extra volume of water lost by this route in the ryegrass ewes is likely to be small. Also, on one occasion (Day 6) when there was little difference in dry matter intake between the two diets (400 g DM for plantain, 439 g DM for ryegrass) so that the respiratory water losses would be similar between the two groups, urine volume remained much higher in plantain ewes (3442 vs 2582 ml) and this difference in urine volume was not offset by the lower faecal water loss in plantain ewes (185 vs 303 ml). The overall difference in water balance (total in minus urine + faecal) on this day being approx. 1105 mL for ryegrass and approx. 383 mL for plantain (i.e. a 722 mL difference in water balance despite presumably similar evaporative water losses in the two groups).

The most striking effect arising from consumption of plantain was observed on the first day. Ewes consuming plantain had very high urine volumes (mean 4805 ml) compared with those on ryegrass (mean 3032 ml) with very little difference between the groups for faecal water (396 and 450 ml, respectively). In the case of ewes consuming plantain this loss of water was far in excess of the water intake and appears to be a profound diuresis. It is likely that homeostatic mechanisms would have commenced operation in these animals to mitigate such effects, which could likely explain the decline in magnitude of the between-group differences over the ensuing six days.

*Osmolarity*

On Day 1 plantain sheep had slightly lower urine osmolarities than plantain sheep (1085 vs 1147 but when multiplied by urine volumes, the total osmols excreted was vastly higher than ryegrass (5266 vs 3497).

However, Day 1 was totally anomalous - the other 6 days osmolarities were much lower in all sheep (typically from 200 to 500) and the total excreted was slightly higher per day in grass sheep (1458) than for plantain (1209).

In spite of this, plantain-fed sheep had higher urine volumes whether you look at Day 1, Days 2-7, or all 7 days.

This suggests plantain is making them urinate water because the extra urine can't be explained by osmotic loading. The lack of osmotic loading suggests sodium is not an important mechanism in this effect. The extra osmols excreted by plantain sheep on Day 1 is likely to be a result of the huge water excretion taking solutes with it and hence the lower osmotic concentration but higher total output that day. Thereafter (Days 2 - 7) osmotic outputs were similar (but higher for ryegrass) but volumes (water) were higher for plantain.

*Urine pH*

*Effect of diet on urine pH.*

On each of the 7 days, the urine pH of plantain-fed sheep was lower than the urine pH of their grass-fed counterparts (Figure 1). The inventors believe that dietary cation-anion difference (DCAD) has a significant effect on urinary pH, with lower DCAD values leading to lower urine pH.

The significant reduction in urine pH would not have been expected to occur concurrently with an increase in urine volume when fed plantain. In particular, for a solution having a given pH it would be expected that the pH of that solution would tend towards neutral as the solution is diluted with water. While the buffering capacity of urine would be expected to withstand some change in pH due to dilution, the results of the osmolarity testing suggest that excreted buffering capacity (based on total dissolved solute excreted) was potentially *reduced* in plantain fed animals compared with grass fed animals.

**Example 1 - Sheep Study IB**

***Experimental***

Animals used in this study were 6 month old Romney × Southdown ewe hoggets. Prior to the study they were grazed on predominantly ryegrass/white clover pasture near Lincoln, Canterbury, New Zealand (Kimihia Research Centre). Two weeks prior to the experiment they were moved to their experimental diets gazed *in situ* and subsequently transported to Johnstone Memorial Laboratory, Lincoln University where they were held indoors for 7 days in individual metabolism crates in one of two rooms.

The sheep were allocated to one of two treatment groups (n = 7 sheep) that were balanced for live weight (average 38 kg) Treatments were either plantain (*Plantago lanceolata, cv* Ceres Tonic) or perennial ryegrass (*Lolium perenne,* cultivars Base and Expo) offered at rate so that each sheep had feed left prior to the following mornings feed. Refused feed from the previous day was collected prior to feeding and weighed, and a sub sample taken for DM analysis. Sheep at access to drinking water from buckets which had known volumes of water from which water consumed was calculated. All procedures involving these animals were approved by the Lincoln University Animal Ethics Committee.

*Urine and faeces*

Urine and faecal matter were collected separately into collection trays and buckets situated under the grating of each metabolism crate. To prevent cross-contamination net separators (shade cloth stretched over a metal frame and fixed underneath the metabolism crates) were used to trap faeces and allow urine to pass through the mesh.

Urine was acidified to prevent nitrogen losses by adding 250 mL of 5% $H_2SO_4$ solution to the collection trays. Daily urine volume for each sheep was measured in a volumetric flask and a 70 mL sample was placed into a polypropylene container and stored in a freezer. The collection trays were rinsed with tap water, returned to each metabolism crate and left until a non-acidified (fresh) sample of (70 mL) urine could be collected. Acid was then placed in the tray for urine collected overnight. Fresh and acidified urine samples were bagged separately in plastic bags and stored frozen for subsequent analyses.

The metabolism crates were cleaned daily to collect all faecal matter and each sheep's daily faecal production was weighed. A small sample was set aside to be freeze dried for subsequent analysis. The rest was divided between two perforated plastic bags which were weighed and placed in 90 °C ovens for 48 h to dry. Drying time depended on the size of the sample, therefore if a sample did not dry in the 25 allocated time it was left for a further 48 h. Once dry, the bags were re-weighed and the dry matter percentage calculated (i.e. dry weight/wet weight × 100). This gave two values for faecal dry matter percentage (DM %) for each sheep's daily output. The mean of these two DM% values was applied to the total faecal weight to determine faecal water content.

This experiment was repeated 3 times with a 1 week period between experiments where sheep returned to the paddock and grazed their experimental diet.

***Results***

Water intake was greater in plantain fed lambs primarily because they consumed more water in their feed, although in the first experiment plantain lambs drank slightly more water. Urine volumes were greater for plantain fed lambs in each of the three experiments but water voided in faecal material was lower in plantain fed lambs (Table 1).

**Table 1**- relationship between diet and water balance in sheep

| Expt | Forage | WaterEaten (ml) | Drink (ml) | Urine (ml) | % urine volume increase | Faecal water (ml) | Balance (ml) |
|---|---|---|---|---|---|---|---|
| 1 | Grass | 5074 | 193 | 3532 | 23% | 562 | 1174 |
| | Tonic | 4990 | 420 | 4349 | | 447 | 617 |
| 2 | Grass | 5578 | 510 | 3992 | 36% | 437 | 1659 |
| | Tonic | 6871 | 308 | 5443 | | 387 | 1377 |
| 3 | Grass | 5354 | 184 | 3796 | 47% | 510 | 1082 |
| | Tonic | 6638 | 195 | 5580 | | 396 | 857 |

***Discussion***

*Urine volume*

In spite of similar volumes of water ingested (experiment 1), ewes consuming plantain had greater urinary volumes than those on ryegrass. These differences in urine volume cannot be explained by faecal loss of water or by respiratory losses from metabolism of the extra dry matter of ryegrass (see below). Thus, it is concluded that the consumption of plantain has produced a diuresis in these sheep.

Where DM% was lower for plantain than for ryegrass (experiments 2 & 3) water intake was higher primarily through water consumed in feed. Although it would be expected that increases in water intake would lead to increases in urine volume, the increases were greater than expected as indicated by the reduction in water balance and therefore continue to demonstrate diuresis in sheep.

The calculation of water balance does not include evaporative water loss (insensible from skin and respiratory losses). Much of the insensible loss would be expected to be very similar for the two groups. However, respiratory water loss is likely to be greater in ryegrass ewes that were metabolising about 200 more g per day of dry matter then the plantain ewes. Nevertheless, the extra volume of water lost by this route in the ryegrass ewes is likely to be small.

**Example 1** - **Sheep study II**

Animals used in this study were 6 month old Romney × Southdown ewe hoggets. Prior to the study they were grazed on predominantly ryegrass/white clover pasture near Lincoln, Canterbury, New Zealand (Kimihia Research Centre) then transported to the Johnstone Memorial Laboratory, Lincoln University where they were held indoors for 3 days in individual metabolism crates without access to drinking water. The sheep were allocated to one of two treatment groups (n = 8) that were balanced for live weight (average 38 kg) and urine production (daily volume) and held in two separate rooms at the Johnstone Memorial Laboratory, with 4 animals from each group per room. Treatments were either plantain (*Plantago lanceolata, cv* Ceres Tonic) or perennial ryegrass (*Lolium perenne,* cultivars Base and Expo) offered at rate so that each sheep consumed 4 litres of water in their feed daily. Each day's individual sheep feed allowance was computed from the dry matter content of the previous day's feed to maintain the constant daily water allocation. Any refused feed was collected the next day and weighed, then discarded. All procedures involving these animals were approved by the Lincoln University Animal Ethics Committee. Urine and faecal matter were collected separately into collection trays and buckets situated under the grating of each metabolism crate. To prevent cross-contamination net separators (shade cloth stretched over a metal frame and fixed underneath the metabolism crates) were used to trap faeces and allow urine to pass through the mesh. The metabolism crates were cleaned daily to collect all faecal matter and each sheep's daily faecal production was weighed. A small sample was set aside to be freeze dried for subsequent analysis. The rest was divided between two perforated plastic bags which were weighed and placed in 90 °C ovens for 48 h to dry. Drying time depended on the size of the sample, therefore if a sample did not dry in the allocated time it was left for a further 48 h. Once dry, the bags were re-weighed and the dry matter percentage calculated (i.e. dry weight/wet weight × 100). This gave two values for faecal dry matter percentage (DM %) for each sheep's daily

output. The mean of these two DM% values was applied to the total faecal weight to determine faecal water content.

This experiment was repeated twice and the results are provided in Table 2.

**Table 2** - relationship between diet and water balance in sheep (% increase shown in parentheses)

| Experiment | Forage | Day 1 | Day 2 | Day 3 | Total |
|---|---|---|---|---|---|
| 1 | Grass | 2888 | 3171 | 2973 | 9031 |
| | Tonic | 3974 (+38%) | 4175 (+32%) | 3378 (+14%) | 11526 (+28%) |
| 2 | Grass | 2563 | 1908 | 1543 | 6013 |
| | Tonic | 3318 (+29%) | 2878 (+51%) | 2238 (+45%) | 8433 (+40%) |

Despite the same intake of water (4000 ml consumed in feed) daily urine volume and total urine volume over 3 days was greater for plantain-fed lambs than ryegrass-fed lambs. Differences in urine volume between treatments was largest in days 1 & 2.

These results continue to demonstrate the diuretic effect of plantain, increasing urine volume from a similar water intake.

## EXAMPLE 2

### Indoor dairy heifer study

***Materials and methods***

*Design and management*

The study was undertaken at Lincoln University Research Dairy Farm, Canterbury, New Zealand under the authority of the Lincoln University Animal Ethics Committee (#296-13). Twelve 8-9 month Holstein-Friesian dairy heifers were used. Heifers grazed on perennial ryegrass/white-clover pasture prior to the study were equally divided into two dietary treatment groups (ryegrass pasture or plantain) based on their liveweight and breeding worth (BW). The study consisted of a 13 day adaptation period and a 3 day N study period. Heifers were given the same daily grazing allowance (on a DM basis), calculated according to the requirement for maintenance × 1.5 during adaptation period. Four days prior to measurement period, heifers were adapted to the stalls where they were housed individually with predesigned urine harness attached.

Freshly cut forage was offered to individual heifer in metabolism stalls at 1000 h and 1300 h, daily. All heifers received the same feed allowance (on a DM basis) during N balance study period, calculated according to the requirement for maintenance × 1.5. Fresh water was available at all times throughout the study.

*Feed and water measurement and analysis*

Intake was measured daily per heifer by weighing offered and refused forage. Forage samples (500 g fresh weight) were collected at feeding and oven-dried at 65 °C to determine DM°/, before grinding through a 1.0-mm sieve (Christy Lab Mill; Suffolk; UK). Forage samples were analyzed by Near Infra-Red Spectroscopy (NIRS systems 6500; feed TECH; New Zealand) for predicted crude protein (CP), organic matter digestibility, acid detergent fibre, neutral detergent fibre, and soluble sugar and starch content. Metabolisable Energy (ME) content was derived from predicted organic matter digestibility on the basis of an *in vitro* cellulase digestibility assay, calibrated against *in vivo* standards.

Daily water intake was measured for each individual heifer daily by recording water volumes removed from the drinking bucket.

*Plasma sampling and analysis*

Two 10 ml blood sample were collected using a Li-heparinized evacuated tube from the jugular vein of each cow at 1400 h daily while in the met stall. Plasma was harvested following centrifugation at 1200 × g for 12 min at 4 °C. At the end of the collection period, plasma samples were pooled per heifer and stored at -20 °C. Plasma urea N (PUN) was quantified using a Daytona RX clinical analyser (Randox; Nishinomiya; Japan).

*Faeces and urine collection and analysis*

Total output of urine from each heifer was collected three times a day at 0930, 1430, and 1930 h using the externally applied urine harness attached with a pipe connector. Urine drained into individual container containing hydrochloric acid placed under the crate to ensure urine pH < 3 in order to prevent N volatilisation.

Three times a day at 0930, 1430, and 1930 h spot urine sample were collected and then stored at -20 °C. Daily faeces was collected from behind the animal at various intervals through the day. Faecal sample was oven-dried at 65 °C to determine DM%. Representative liquid urine and oven-dried faeces from five days bulked samples per heifer was sub sampled and measured for N concentration using a Variomax CN Analyser (Elementar Analysensysteme GmbH; Hanau; Germany).

**Results**

*Water balance*

Water intake from feed was higher for plantain fed heifers due to the lower DM% (or greater water content of plantain). Water intake from drinking was lower for plantain but overall heifers consuming plantain consumed more water and produced more urine. Greater urine production is probably a result of the greater water intake but also the diuretic affect recently demonstrated.

**Table 3** - relationship between diet and water balance in cows (% increase shown in parentheses)

| | **Pasture** | **Plantain** |
|---|---|---|
| DMI (kg/day) | 4.90 | 5.23 |
| DM% | 21.5 | 15.1 |
| Feed water (l/day) | 17.9 | 29.3 |
| Drink water (l/day) | 7.5 | 4.7 |
| Total water intake (l/day) | 25.4 | 34.0 |
| Urine (l/day) | 20.9 | 33.4 (+60%) |

**EXAMPLE 3**

**Outdoor sheep study**

***Urine pH study***

*Sheep*

The effects of grazing different forages on the urine pH of lambs was studied. Example 1 showed differences in urine pH between lambs offered plantain or perennial ryegrass.

This study aimed to repeat this earlier work but also to include a greater array of forages and to measure animal grazing in the field as opposed to being housed indoors. Animals used in this study were lambs (run 1) or hoggets (run 2). Ten lambs/hoggets were randomly allocated to each grazing treatment from a larger (n=200) mob that had been grazing ryegrass pastures. In run 1, ryegrass, chicory, red clover, forage brassica, Lucerne, Tonic plantain, PG742 plantain and WDA+ plantain were used as grazing treatments. In run 2, ryegrass, chicory, red clover Tonic plantain, PG742 plantain and WDA+ plantain were used as grazing treatments. Sheep were set stocked on their grazing treatments with enough forage to allow *ad libitum* intake. Urine samples were taken on Day 0 and at various period after this and tested for pH.

Urine samples were collected by temporary asphyxia (Lincoln University SOP) and this procedure was approved by the Lincoln University Animal Ethics Committee (Appln 658 & 2016-29). Urine pH was determined by a hand-held pH probe calibrated between groups using a buffered standard of known pH (7). Forage samples were collected during grazing to determine DCAD.

*Run 1*

Urine pH was measured over 5 days and is presented in Table 4. Although urine pH generally increased over the period (from 6.8 to 7.8), differences between forages were subtle. Plantain lines (PG742, WDA+ and Tonic) tended to be lower than other forages by 0.3- 0.5 pH units from day 1 onward.

**Table 4.** *Urine pH from lambs (n=10/treatment) grazing forages (including 3 plantain lines) in situ over five days*

| Treatment | Day 0 | Day 1 | Day 2 | Day 3 | Day 4 | Day 5 |
|---|---|---|---|---|---|---|
| Ryegrass | 6.5 | 6.3 | 6.8 | 7.7 | 7.8 | 7.9 |
| Chicory | 7.1 | 7.8 | 7.6 | 7.9 | 7.7 | 7.7 |
| Red clover | 6.7 | 7.6 | 7.8 | 8.0 | 8.0 | 8.1 |
| Brassica | 6.8 | 7.9 | 7.8 | 7.9 | 7.7 | 8.1 |
| Lucerne | 6.8 | 7.8 | 7.9 | 7.9 | 7.9 | 8.0 |
| PG 742 plantain | 7.0 | 7.0 | 6.2 | 7.6 | 7.3 | 7.6 |
| WDA+ plantain | 6.9 | 7.5 | 7.5 | 7.5 | 7.5 | 7.5 |
| Tonic plantain | 6.4 | 6.7 | 6.5 | 7.2 | 7.3 | 7.7 |

*Run 2*

In run 2, urine pH was measured at Day 0, 3 and 8 after being introduced to the forage treatments and is presented in Table 5.

**Table 5.** *Urine pH from lambs (n=11/treatment) grazing forages (including 3 plantain lines) in situ over eight days with corresponding DCAD.*

| Treatment | Day 0 | Day 3 | Day 8 | Day 15 | DCAD |
|---|---|---|---|---|---|
| Ryegrass | 8.1 | 6.9 | 7.5 | 7.4 | 231 |
| Red clover | 8.2 | 7.2 | 8.2 | 8.2 | |
| Chicory | 8.1 | 7.1 | 7.1 | 7.4 | |
| WDA+ | 8.0 | 6.1 | 5.7 | 5.9 | |
| Tonic | 8.0 | 5.5 | 5.5 | 5.1 | -228 |
| PG742 | 8.0 | 5.3 | 5.2 | 5.1 | -189 |

There was a decline in urine pH in ryegrass and chicory treatments from Day 0 to either day 3 or Day 8 and some variation in urine pH with red clover over time but urine pH for these forages did not drop below 7 at any time. However, for all plantain lines, there was a significant drop in urine pH from pretreatment measurements to day 3 which was maintained at day 8. The pH of urine from lambs grazing PG 742 reached 5.2 on day 8, more than 2 pH units lower than ryegrass at the same time. The decrease was not so large for the WDA+ line. Corresponding DCAD values for the plantain and ryegrass forages also showed large differences.

This data highlights the potential for plantain to reduce urine pH and the extent of this reduction may differ between plantain lines. Although there seems to be some inconsistencies between run 1 and 2 in the effect of plantain on urine pH, some trial management might help explain this. Prior to Run 2, a base fertiliser was applied (300 kg/ha Cropmaster 15) which contained nitrogen (15%), phosphorus (10%), potassium (10%) and sulphur (8%). Although potassium applications would have a positive effect on DCAD, sulphur applications have a negative effect which is more than twice as great per kg applied. The difference in urine pH from plantain between Run 1 and 2 would appear to be related to the availability of sulphur and its effect on DCAD. DCAD values less than 10 (or even negative) and therefore low urine pH are possible on plantain stands (particularly Tonic and Tonic derivatives- PG742) where sulphur availability is not limiting. Accordingly, Run 2 demonstrates that through modulation of the DCAD value of the plantain, such as through fertiliser application, it is possible to modulate the reduction in urine pH of the animal.

No admission is made that any reference constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents form part of the common general knowledge in the art, in New Zealand or in any other country.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

Where in the foregoing description reference has been made to integers or components having known equivalents thereof, those integers are herein incorporated as if individually set forth.

Aspects of the present invention have been described by way of example only and it should be appreciated that modifications and additions may be made thereto without departing from the scope thereof.

## Claims

1. A process of screening a population of plants of the genus *Plantago* for use in increasing the urine volume of an animal, the process including the steps of:

a. measuring the DCAD of each of the population of plants;
b. selecting one or more of the population of plants having DCAD less than 10 meq/100 g DM.

2. The process of claim 1 including the step of selecting one or more of the population of plants having DCAD below 0 meq/100 g DM.

3. A process of screening a population of plants of the genus *Plantago* for use in reducing the pH of the urine of an animal, the process including the steps of:

a. measuring the DCAD of each of the population of plants;
b. selecting one or more of the population of plants having DCAD less than 10 meq/100 g DM.

4. The process of claim 3 including the step of selecting one or more of the population of plants having DCAD below 0 meq/100 g DM.

5. Animal feed including plant material derived from a plant of the genus *Plantago,* wherein the plant has been selected from a population of plants of the genus *Plantago* by a process including the steps of:

a. measuring the DCAD of each of the population of plants;
b. selecting one or more of the population of plants having DCAD less than 0 meq/100 g DM.

6. The animal feed according to claim 5 wherein the plant of the genus *Plantago* is *Plantago lanceolata.*

7. The animal feed according to claim 6 wherein the *Plantago lanceolata* is characterized as providing winter growth.

8. A population of plants of the genus *Plantago* having DCAD below 0 meq/100 g DM.

9. Use of plant material derived from a plant of the genus *Plantago* to reduce the osmolarity of urine of an animal.

10. The use according to claim 9 wherein the plant of the genus *Plantago* is *Plantago lanceolata.*

11. The use according to claim 10 wherein the *Plantago lanceolata* is characterized as providing winter growth.

12. Use of plant material derived from a plant of the genus *Plantago* to dilute the urine of an animal.

13. The use according to claim 12 wherein the plant of the genus *Plantago* is *Plantago lanceolata.*

14. The use according to claim 13 wherein the *Plantago lanceolata* is characterized as providing winter growth.

**Patentansprüche**

1. Verfahren zum Screenen einer Population von Pflanzen der Gattung *Plantago* zur Verwendung bei der Steigerung des Harnvolumens eines Tieres, wobei das Verfahren die folgenden Schritte umfasst:

a. Messen des DCAD jeder Population von Pflanzen;
b. Auswählen einer oder mehrerer Populationen von Pflanzen, die einen DCAD geringer als 10 meq/100 g DM aufweisen.

2. Verfahren nach Anspruch 1, das den Schritt des Auswählens einer oder mehrerer der Populationen von Pflanzen umfasst, die einen DCAD unter 0 meq/100 g DM aufweisen.

3. Verfahren zum Screenen einer Population von Pflanzen der Gattung *Plantago* zur Verwendung bei der Reduzierung des pH-Werts des Harns eines Tieres, wobei das Verfahren die folgenden Schritte umfasst:

a. Messen des DCAD jeder Population von Pflanzen;
b. Auswählen einer oder mehrerer Populationen von Pflanzen, die einen DCAD geringer als 10 meq/100 g DM aufweisen.

4. Verfahren nach Anspruch 3, das den Schritt des Auswählens einer oder mehrerer der Populationen von Pflanzen, die einen DCAD unter 0 meq/100 g DM aufweisen, umfasst.

5. Tierfutter, das Pflanzenmaterial umfasst, das von einer Pflanze der Gattung *Plantago* gewonnen wird, wobei die Pflanze aus einer Population von Pflanzen der Gattung *Plantago* durch ein Verfahren mit den folgenden Schritten ausgewählt wird:

   a. Messen des DCAD jeder Population von Pflanzen;
   b. Auswählen einer oder mehrerer Populationen von Pflanzen, die einen DCAD geringer als 0 meq/100 g DM aufweisen.

6. Tierfutter nach Anspruch 5, wobei die Pflanze der Gattung *Plantago Plantago lanceolata* ist.

7. Tierfutter nach Anspruch 6, wobei die Gattung *Plantago lanceolata* **dadurch gekennzeichnet ist, dass** sie Winterwachstum bereitstellt.

8. Population von Pflanzen der Gattung *Plantago,* die einen DCAD unter 0 meq/100 g DM aufweist.

9. Verwendung von Pflanzenmaterial, das aus einer Pflanze der Gattung *Plantago* gewonnen wird, um die Osmolarität von Urin eines Tieres zu verringern.

10. Verwendung nach Anspruch 9, wobei die Pflanze der Gattung *Plantago Plantago lanceolata* ist.

11. Verwendung nach Anspruch 10, wobei die Gattung *Plantago lanceolata* **dadurch gekennzeichnet ist, dass** sie Winterwachstum bereitstellt.

12. Verwendung von Pflanzenmaterial, das von einer Pflanze der Gattung *Plantago* gewonnen wird, um den Urin eines Tiers zu verdünnen.

13. Verwendung nach Anspruch 12, wobei die Pflanze der Gattung *Plantago Plantago lanceolata* ist.

14. Verwendung nach Anspruch 13, wobei die Gattung *Plantago lanceolata* **dadurch gekennzeichnet ist, dass** sie Winterwachstum bereitstellt.

## Revendications

1. Procédé de criblage d'une population de plantes du genre *Plantago* pour une utilisation dans l'augmentation du volume d'urine d'un animal, le procédé comprenant les étapes consistant à :

   a. mesurer la DCAD de chacune de la population de plantes ;
   b. sélectionner une ou plusieurs de la population de plantes ayant une DCAD inférieure à 10 meq/100 g de matière sèche (MS).

2. Procédé selon la revendication 1, comprenant l'étape consistant à sélectionner une ou plusieurs parmi la population de plantes ayant une DCAD inférieure à 0 meq/100 g de MS.

3. Procédé de criblage d'une population de plantes du genre *Plantago* pour une utilisation dans la réduction du pH de l'urine d'un animal, le procédé comprenant les étapes consistant à :

   a. mesurer la DCAD de chacune de la population de plantes ;
   b. sélectionner une ou plusieurs de la population de plantes ayant une DCAD inférieure à 10 meq/100 g de MS.

4. Procédé selon la revendication 3, comprenant l'étape consistant à sélectionner une ou plusieurs parmi la population de plantes ayant une DCAD inférieure à 0 meq/100 g de MS.

5. Alimentation animale comprenant du matériel végétal dérivé d'une plante du genre *Plantago,* dans laquelle la plante a été sélectionnée à partir d'une population de plantes du genre *Plantago* par un procédé comprenant les étapes

consistant à :

a. mesurer la DCAD de chacune de la population de plantes ;
b. sélectionner une ou plusieurs de la population de plantes ayant une DCAD inférieure à 0 meq/100 g de MS.

**6.** Alimentation animale selon la revendication 5, dans laquelle la plante du genre *Plantago* est *Plantago lanceolata.*

**7.** Alimentation animale selon la revendication 6, dans laquelle la plante *Plantago lanceolata* est **caractérisée en ce qu'**elle fournit une croissance hivernale.

**8.** Population de plantes du genre *Plantago* ayant une DCAD inférieure à 0 meq/100 g de MS.

**9.** Utilisation de matériel végétal dérivé d'une plante du genre *Plantago* pour réduire l'osmolarité d'urine d'un animal.

**10.** Utilisation selon la revendication 9, dans laquelle la plante du genre *Plantago* est *Plantago lanceolata.*

**11.** Utilisation selon la revendication 10, dans laquelle la plante *Plantago lanceolata* est **caractérisée en ce qu'**elle fournit une croissance hivernale.

**12.** Utilisation de matériel végétal dérivé d'une plante du genre *Plantago* pour diluer l'urine d'un animal.

**13.** Utilisation selon la revendication 12, dans laquelle la plante du genre *Plantago* est *Plantago lanceolata.*

**14.** Utilisation selon la revendication 13, dans laquelle la plante *Plantago lanceolata* est **caractérisée en ce qu'**elle fournit une croissance hivernale.

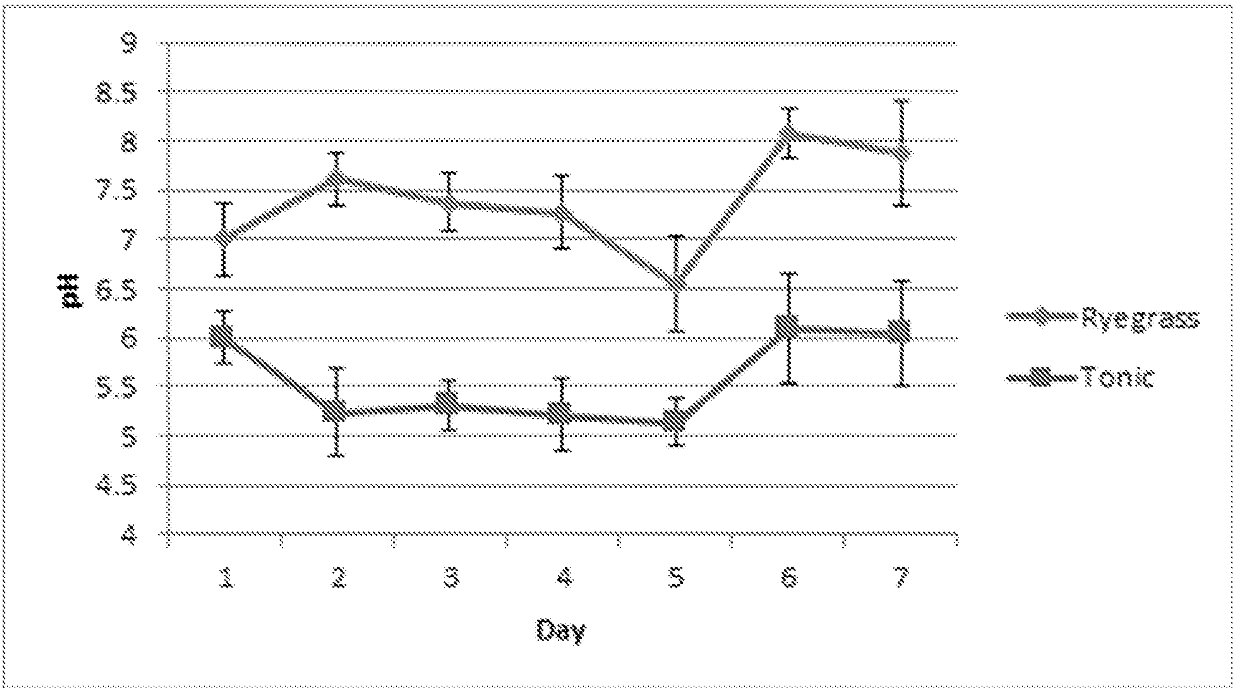


Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JEAN-LUC RIOND.** Animal Nutrition and Acid Base Balance. *European Journal of Nutrition,* 2001, vol. 40, 245-254
- Periparturient responses of cows fed varying dietary cation-anion differences and calcium contents prepartum. **RODRIGUEZ-SUAREZ, L.A.** PhD dissertation. Michigan State University, 1998
- The use of dietary cation anion difference for the reduction of urine pH in goats. **JONES, M.L.** MSc disseration. Oklahoma State University, 2006
- **H.G. JUDSON ; R. MCANULTY ; R. SEDCOLE.** *Proceedings of the New Zealand Grassland Association,* 2009, vol. 71, 201-205